(19) Europäisches Patentamt
European Patent Office
Office européen des brevets

(11) Veröffentlichungsnummer: **0 105 168**
**B1**

(12) # EUROPÄISCHE PATENTSCHRIFT

(45) Veröffentlichungstag der Patentschrift: **26.02.86**

(51) Int. Cl.⁴: **C 08 G 18/79**, C 07 D 229/00

(21) Anmeldenummer: **83108253.2**

(22) Anmeldetag: **22.08.83**

(54) **Aromatische Uretdion-di-harnstoff-diamine, Verfahren zu ihrer Herstellung und ihre Verwendung bei der Polyurethansynthese.**

(30) Priorität: **03.09.82 DE 3232736**

(43) Veröffentlichungstag der Anmeldung: **11.04.84 Patentblatt 84/15**

(45) Bekanntmachung des Hinweises auf die Patenterteilung: **26.02.86 Patentblatt 86/9**

(84) Benannte Vertragsstaaten: **BE DE FR GB IT NL**

(56) Entgegenhaltungen:
**AT - B - 349 031**
**DE - A - 2 044 838**
**DE - A - 2 221 170**

(73) Patentinhaber: **BAYER AG, Konzernverwaltung RP Patentabteilung, D-5090 Leverkusen 1 Bayerwerk (DE)**

(72) Erfinder: **Hess, Heinrich, Dr., Auf der Griesse 48, D-5090 Leverkusen 1 (DE)**
Erfinder: **Grögler, Gerhard, Dr., von-Diergardt-Strasse 46, D-5090 Leverkusen 1 (DE)**
Erfinder: **Kopp, Richard, Dr., Wolfskaul 12, D-5000 Köln 80 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der Erfindung sind neue, Uretdion- und Harnstoffgruppen-haltige aromatische Diamine

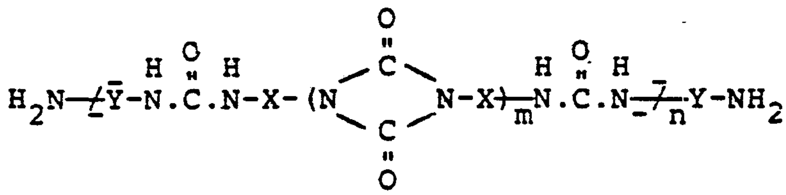

wobei
X aromatische Reste mit Molmassen von 76 bis 499,
Y aromatische Reste wie X oder Reste

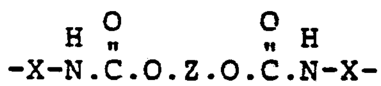

eines höhermolekularen Diamins darstellen, wo
Z der Rest von höhermolekularen und/oder niedermolekularen Dihydroxyverbindungen HO-Z-OH ist,
n der mittlere Vorverlängerungsgrad mit Zahlen zwischen 1 und 3 ist und
m der mittlere Uretdionisierungsgrad mit Zahlen zwischen 1 und 10 ist,
wobei Diamine ausgeschlossen sind, bei welchen X der 2,4'-Diphenylsulfid-Rest ist, wenn das Diamin $H_2N$-Y-$NH_2$ Molmassen von ≥ 500 besitzt.

Weiterer Erfindungsgegenstand ist ein Herstellungsverfahren dieser Diamine aus Uretdiondiisocyanaten und überschüssigen aromatischen Diaminen bzw. höhermolekularen Verbindungen mit aromatischen Aminogruppen, sowie ihre Verwendung als Aufbaukomponente von Polyadditionsprodukten, vorzugsweise als Diamin-Vernetzer mit starren, gegebenenfalls vernetzbaren Uretdionsegmenten beim Aufbau von polyurethanen.

Uretdiondiamine, d.h. aromatische Diamine mit Uretdiongruppen im selben Molekül mit der allgemeinen Formel

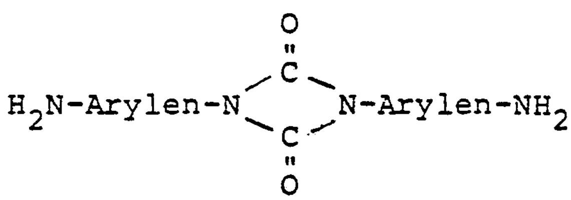

I

sind schon bekannt und können zum Einbau von Uretdiongruppen in heiß zu härtende polyurethane (DE-OS 2 044 838) oder zur Herstellung waschechter Farben auf Cellulosefasern (US-pS 2 643 250) verwendet werden. Im letzteren Fall wird das Uretdiondiamin zuerst über die Uretdiongruppe an die Cellulosefaser gebunden und die Aminogruppen werden anschließend diazotiert.

Die Herstellungsweise von aromatischen Uretdiondiaminen durch Nitrierung von Uretdionen und anschließende Hydrierung entsprechend US-pS 2 643 250 ist jedoch recht aufwendig.

Aliphatische Uretdiongruppen-haltige Diamine sind praktisch nicht stabil. So reagiert dimeres Toluylendiisocyanat mit aliphatischen Aminen bereits bei Temperaturen unter 50°C unter Aufspaltung des Uretdionrings und unter Harnstoff- bzw. Biuret-Bildung (JACS 75 (1953) 8. 5439 und Can. J. Chem. 40 (1962), S. 935). Diese chemische Eigenart der Uretdiongruppierung wird bei der Vernetzung uretdiongruppenhaltiger polyurethane durch Einwirkung von aliphatischen Diaminen genutzt, z.B. bei der Vernetzungsreaktion des Uretdionrings mit Ethylendiamin zur Herstellung von vernetzten polyurethan-Filmen und -Fasern (DE-OS 2 044 838). Selbst die weniger reaktiven aromatischen Amine können den Uretdionring in polyurethanen in Dimethylformamid- oder Dimethylsulfoxid-Lösung aufspalten.

In den DE-OS 2 941 051, 2 842 805 und 1 570 548 werden zur Herstellung von polyurethan-Einkomponentenmischungen neben dimerem Toluylendiisocyanat auch aromatische Diamine als Kettenverlängerer mitverwendet.

Definierte Addukte aus dimerem Toluylendiisocyanat mit Überschüssen an Diaminen sind dort jedoch nicht beschrieben.

Es wurde nun eine einfache Möglichkeit aufgefunden, zu aromatischen Uretdion-diharnstoff-diaminen zu gelangen, indem man 1 Mol eines Uretdion-diisocyanats mit 2 Mol eines aromatischen Diamins umsetzt, wobei ein mit Harnstoffgruppen modifiziertes Uretdion-diamin entsteht:

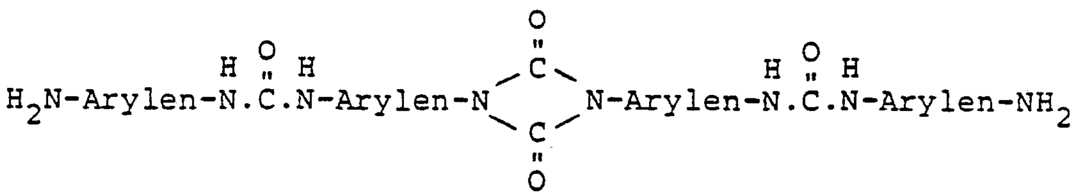

(Arylen = X; Molmasse 76-499). ("Dimer-diamin")

II

Es ist überraschend, daß bei dieser Umsetzung definierte produkte entstehen, denn der Uretdionring hätte auch mit dem überschüssigen Diamin zum Biuret oder gar weiter gespalten werden können.

Die niedermolekularen Addukte aus 1 Mol aromatischem Uretdion-diisocyanat (Formel IV)

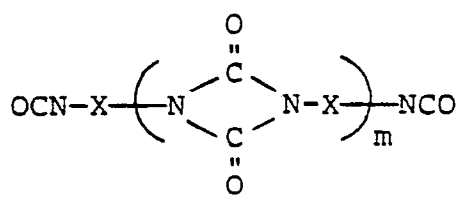

IV

und überschüssigen Mengen, z.B. 2 Mol aromatischem Diamin (V)

$H_2N\text{-}Y\text{-}NH_2$ V

sind leicht herzustellen. Sie brauchen nach der Herstellung und Trocknung nicht gemahlen zu werden, da sie in Form sehr feiner pulver von z.B. 1 bis 3 µm anfallen. Sie erweisen sich bis weit über 100°C stabil gegenüber Uretdionspaltung.

Gegenstand der Erfindung sind somit neuartige aromatische Uretdion-diharnstoff-diamine der Formel:

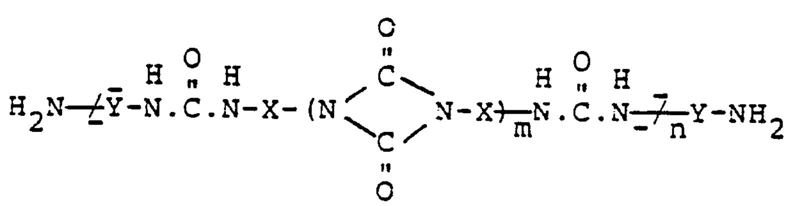

("Dimer-diamine")

worin

X aromatische Reste mit Molmassen von 76 bis 499,

Y aromatische Reste wie X oder Reste

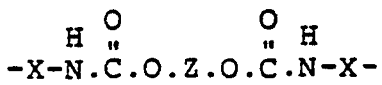

eines höhermolekularen Diamins darstellen, wo
Z der Rest von höhermolekularen und/oder niedermolekularen Dihydroxyverbindungen HO-Z-OH ist,
n der mittlere Vorverlängerungsgrad mit Zahlen zwischen 1 und 3, vorzugsweise zwischen 1 und 2 ist, und
m der mittlere Uretdionisierungsgras mit Zahlen zwischen 1 und 10, bevorzugt zwischen 1 und 2, besonders bevorzugt jedoch 1, ist und
wobei Diamine ausgeschlossen sind, bei welchen X der 2,4 -Diphenylsulfid-Rest ist, wenn das Diamin $H_2N$-Y-$NH_2$ Molmassen von ⩾ 500 besitzt.

Weiterer Erfindungsgegenstand ist ein Verfahren zur Herstellung von aromatischen Uretdion-diharnstoffdiaminen der Formel

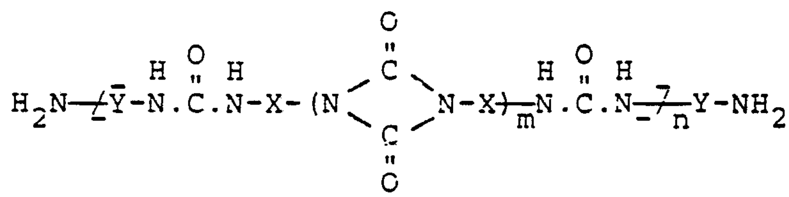

in feinteiliger, gegebenenfalls suspendierter Form durch Vermischen einer Lösung oder Suspension eines aromatischen Diamins

$H_2N$ - Y - $NH_2$

in einem gegenüber Isocyanaten inerten Lösungsmittel, Weichmacher oder einem höhermolekularen polyol mit einem feinteiligen, gegebenenfalls in einem gegenüber Isocyanaten inerten Lösungsmittel, Weichmacher oder einem hdhermolekularen polyol suspendierten Uretdion-diisocyanat

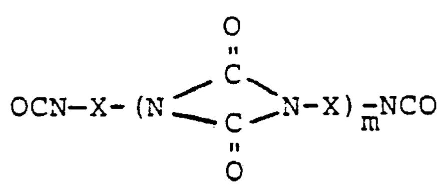

wobei das Molverhältnis von Diamin zu Diisocyanat ⩾ 1,8: 1, vorzugsweise 1,8 bis 2,3, ist und
X aromatische Reste mit Molmassen von 76 bis 499, und
Y aromatische Reste wie X oder
Reste

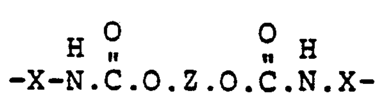

darstellen, wo
Z der Rest von höhermolekularen und/oder niedermolekularen Dihydroxyverbindungen HO-Z-OH ist,
n der mittlere Vorverlängerungsgrad mit Zahlen zwischen 1 und 3 ist und
m der mittlere Uretdionisierungsgrad mit Zahlen zwischen 1 und 10 ist,

wobei die Herstellung solcher aromatischer Uretdiondiharnstoff-diamine ausgeschlossen ist, bei welchen X der 2,4'-Diphenylsulfidrest ist und das Diamin $H_2N$-Y-$NH_2$ Molmassen von ⩾ 500 aufweist.

Weiterer Erfindungsgegenstand ist die Verwendung der feinteiligen Uretdion-diharnstoff-diamine, gegebenenfalls suspendiert in höhermolekularen polyhydroxylverbindungen, als Aufbaukomponente zur Herstellung von Harnstoffgruppen haltigen, hochmolekularen, gegebenenfalls über die Uretdiongruppen vernetzbaren polyadditionsprodukten. Erfindungsgegenstand ferner ist die Verwendung der Uretdion-diharnstoff-diamine, dadurch gekennzeichnet, daß man die feinteiligen Uretdion-diharnstoff-diamine gegebenenfalls suspendiert in höhermolekularen Polyhydroxylverbindungen zusammen mit NCO-prepolymeren aus polyolen und überschüssigen polyisocyanaten und/oder zusammen mit Polyisocyanaten, sowie gegebenenfalls weiteren höhermolekularen Polyhydroxylverbindungen der Molmasse 400 bis 10 000 und gegebenenfalls niedermolekularen Kettenverlängerungsmitteln, besonders Diolen oder aromatischen Diaminen der Molmasse 62 bis J99 bei Temperaturen oberhalb 100°C und einem Äquivalentverhältnis zwischen NCO-Gruppen und der Summe von OH- und NH -Gruppen zwischen 0,9 und 1,4 umsetzt und gegebenenfalls bei Anwesenheit überschüssiger OH- oder $NH_2$-Gruppen bei Temperaturen oberhalb 140°C vernetzt. Weiterer Erfindungsgegenstand ist die Verwendung der Urethanharnstoffdiamine dadurch gekennzeichnet, daß man Umsetzungsprodukte von langkettigen Diaminen $H_2N$-Y-$NH_2$ mit einer Molmasse ⩾ 500 mit Uretdiondiisocyanaten, gegebenenfalls in Anwesenheit von weiteren, niedermolekularen aromatischen Diaminen bei Temperaturen oberhalb 140°C als selbstvernetzendes Einkomponentensystem einsetzt.

Nach dem Verfahren zur Herstellung der Dimer-diamine entsteht eine thixotrope, feinteilige Suspension, die man noch mehrere Stunden bei Raumtemperatur bis 110°C, vorzugsweise 40 bis 50°C nachrührt. Da auch bei einem Molverhältnis von 2:1 eine geringe "Vorverlängerung" zwischen Diisocyanat und Diamin eintreten kann, bleibt in der flüssigen Lösungsmittelphase etwas überschüssiges aromatisches Diamin in den meisten Fällen gelöst. Man wäscht diese mit organischen Lösungsmitteln aus, saugt das Dimer-diamin ab, und trocknet das produkt. Diese Dimer-diamine weisen im IR-Spektrum keinen freien NCO-Gehalt mehr auf. Die Biuret-Bande ist nicht oder höchstens als schwache Schulter erkennbar. Die Uretdion-Bande bleibt auch nach Erhitzen auf 110°C erhalten. Die Herstellung der produkte ist natürlich auch möglich, indem man das Uretdion-diisocyanat in Lösung oder Suspension vorlegt und das Amin zugibt.

Gegenstand der Erfindung ist auch die Verwendung der verfahrensgemäß erhältlichen Uretdion-diharnstoff-diamine (Dimer-diamine), gegebenenfalls suspendiert in höhermolekularen polyhydroxylverbindungen, als Aufbaukomponente zur Herstellung von Harnstoffgruppen aufweisenden, hochmolekularen, gegebenenfalls über die Uretdiongruppierung vernetzbaren polyurethanen. 8o können die "Dimer-diamine" zur Diaminvernetzung nach dem polyisocyanat-polyadditionsverfahren, vorzugsweise in fester, feinpulverisierter, heterogener Form eingesetzt werden. Sie werden so als pulver, vorzugsweise des Korngrößenbereichs 0,01 bis 10 µm, besonders bevorzugt 0,1 bis 3 µm, in einem polyol angeteigt oder suspendiert und zusammen mit NCO-prepolymeren und/oder polyisocyanaten und gegebenenfalls weiteren Kettenverlängerungsmitteln der Molmasse 62 bis 399, vorzugsweise niedermolekularen polyolen oder aromatischen Diaminen, zu Elastomeren mit überraschend hohem Eigenschaftsniveau verarbeitet. Das System ist dabei lagerstabil bei Raumtemperatur oder wenig erhöhten Temperaturen und besitzt auch bei höheren Temperaturen eine gute Fließfähigkeit und lange Verarbeitungszeit ("Topfzeit"). Bei den Elastomerendprodukten ist der kleine Druckverformungsrest bei trotzdem hoher Zugfestigkeit besonders hervorzuheben. Heizt man bei 110°C zur polyurethanbildung aus, so bleibt während des Ausheizens der Uretdionring weitgehend erhalten (Nachweis durch IR-Spektren). Bei nach höheren Ausheiztemperaturen vermag der Uretdionring unter teilweiser weitervernetzung des Elastomeren weiterzureagieren.

Während die problematik bei der heterogenen Diaminvernetzung im allgemeinen darin liegt, daß ein bei Raumtemperatur festes und schwer lösliches Diamin sich zwar in der Hitze lösen soll, aber trotzdem nur so langsam mit den freien NCO-Gruppen abreagiert, daß sich um die Feststoffteilchen herum nicht sofort eine Schicht Harnstoff bildet, sind überraschenderweise die erfindungsgemäßen "Dimer-diamine" trotz ihrer auch bei höheren Temperatur nur geringen Löslichkeit für die heterogene Diaminvernetzung sehr gut geeignet.

Die erfindungsgemäßen "Dimer-diamine" werden vorzugsweise in Form von Abmischungen der Dimer-diamine mit den höhermolekularen Verbindungen mit 2 oder mehreren, gegenüber Isocyanat reaktiven Gruppen, z.B. höhermolekularen polyether- oder polyester-polyolen oder polyether- oder polyester-polyaminen in Form von Suspensionen oder Pasten eingesetzt. Diese Abmischungen werden anstelle der reinen Uretdion-diharnstoff-diamine dann verwendet, wenn die Mischung der reinen Amine mit dem NCO-prepolymer wegen der zu großen Volumenverhältnisse zwischen Diamin und NCO-prepolymer nicht mehr praktikabel gießbar ist. Die Abmischungen können hergestellt werden, indem man die erfindungsgemäßen Dimer-diamine mit einem hochtcurigen Mischgerät in das höhermolekulare Polyol oder Polyamin einarbeitet. Man kann die erfindungsgemäßen Dimer-diamine natürlich auch ohne Umweg direkt im Polyol erzeugen, indem man im Polyol das aromatische Diamin ($H_2N$-Y-$NH_2$) löst und mit dem Uretdion-diisocyanat (das dimere Diisocyanat) vermischt. Der Vorteil dieser einstufigen Methode liegt darin, daß beim Vermischen der Polyurethankomponenten keine inhomogenen Agglomerate auftreten können. Derartige Mischungen, sind bei etwa 80 bis 100° gut fließfähig.

Beim Gießprozeß in Formen wird die Abmischung aus dem erfindungsgemäßen Dimer-diamin und dem Polyol oder polyamin ca. 20 bis 60 min bei einer Temperatur von 50 bis 100°C im Vakuum entgast, mit dem gegebenenfalls etwa 10 bis 30 min bei 80 bis 120°C entgasten NCOprepolymer oder geschmolzenen Polyisocyanat vermischt und bei einer Temperatur von 100 bis 150°C, vorzugsweise 110 bis 120°C zur Umsetzung gebracht und

nachgeheizt.

Statt eines Formgießprozesses kann das Gemisch auch auf Standard-Injektionsmaschinen, vorzugsweise bei höheren Temperaturen wie 140 bis 200° C, verarbeitet werden.

Das Äquivalentverhältnis zwischen NCO-Gruppen und der Summe von OH- und NH -Gruppen kann dabei zwischen 0,9 und 1,4, vorzugsweise zwischen 1,0 und 1,2 liegen. wenn man Uretdionringe zur Reaktion bringt, ist auch der Uretdionring mit in die Berechnung der vorhandenen NCO-Gruppen einzubeziehen. Bei unterschüssigen Mengen an OH- und $NH_2$-Gruppen kann bei hinreichend hohen Temperaturen (z.B. > 140° C) gegebenenfalls eine Selbstvernetzung des Elastomers über die Uretdiongruppen eintreten.

Selbstverständlich können die erfindungsgemäßen Dimerdiamine auch in Abmischung mit bekannten flüssigen oder leicht löslichen aromatischen Di- oder polyaminen verwendet werden. Mit derartigen Mischungen hergestellte Polyetherpolyurethane besitzen ein deutlich höheres mechanisches Werteniveau als Vergleichselastomere, die alleine mit dem flüssigen oder leicht löslichen Vernetzeramin umgesetzt wurden (vgl. Beispiele 28 und 29). Weiterhin stellen gegebenenfalls die Dimer-diamine selbstvernetzende Einkomponentensysteme dar, wenn man sie auf so hohe Temperaturen erwärmt, daß der Uretdionring mit den aromatischen NH -Gruppen (zu Biureten) reagiert (z.B. bei Temperaturen >140° C). Bevorzugt werden diese Einkomponentensysteme durch Reaktion von einem Uretdiondiisocyanat mit einem langkettigen und einem kurzkettigen Diamin hergestellt, um Elastomere zu erhalten. Werden Dimerdiamine mit einem Wert von m > 1 eingesetzt, kann auch weiteres polyol oder polyamin in der Reaktionsmischung zugesetzt werden.

Die als Diamine mit besonders starrem Aufbau verwendeten, erfindungsgemäßen Dimer-diamine entsprechen im wesentlichen einer Zusammensetzung, in der X und Y aromatische Reste mit Molmassen von 76 bis 499 darstellen, wie z.B. 1,3- oder 1,4-Phenylenreste, 2,4- und/oder 2,6-Toluylen-reste, 3,5-Diethyl-2,4-diamino-toluylenreste, 3,5-Diethyl-2,6-diamino-toluylenreste, 4,4'-Diaminodiphenyl-methanreste, in 3- und/oder 5-Stellungen di- und tetraalkylierte Diphenylmethanreste, 4,4'- und/oder 2,4'-Diphenylsulfidreste, 3,3'-Dichlor-4,4'-diphenylmethanreste, 1,5-Naphthylenreste, 3,3'-4,4'-Diphenylreste, 4,4'-Diphenylsulfonreste, und andere bekannte Reste aromatischer Diisocyanate oder aromatische Diamine wie diese in der polyurethanchemie bekannt sind. Wie bereits ausgeführt, ergeben die erfindungsgemäßen Dimer-diamine mit Y = X hochschmelzende, starre Hartsegmente in den polyurethanelastomeren.

Bevorzugt sind als Reste X der 2,4-Toluylenrest, der 4,4'-Diphenylmethanrest und/oder der 2,4'-Diphenylsulfidrest, ganz besonders aber der 2,4-Toluylenrest.

Werden aber solche erfindungsgemäßen Dimer-diamine hergestellt, bei denen der Rest Y der Rest eines höhermolekularen diprimären Diamins $H_2N\text{-}Y\text{-}NH_2$ mit einer Molmasse von > 500 bis 10 000, vorzugsweise 1000 bis 6000 ist, und der Rest Y der Formel

$$-X-\overset{H}{N}.\overset{O}{\overset{\|}{C}}.O\ .\ Z\ .\ O.\overset{O}{\overset{\|}{C}}.\overset{H}{N}-X-$$

entspricht, in der Z der Rest von höhermolekularen und/oder niedermolekularen Dihydroxyverbindungen HO-Z-OH ist, so sind diese Diamine nicht mehr so extrem hochschmelzend und je nach Länge des Restes Z, der z.B. höhermolekulare Dihydroxypolyetherreste oder Dihydroxypolyesterreste darstellen kann, elastifiziert bzw. weniger starr. Mit solchen Dimer-diaminen können Polyurethane aufgebaut werden, wo man weniger Anteile an höhermolekularen Polyolen oder Polyaminen benutzt, wobei diese höhermolekularen Polyole oder Polyamine zur Elastifizierung des polyurethans üblicherweise beitragen (sogenannte Weichsegmente). In besonderen Fällen können derartige Dimer-diamine ausschließlich mit Diisocyanaten zu Polyurethanen umgesetzt werden, bzw. im Extremfall bei sehr hohen Temperaturen zu Polyurethanen selbstvernetzt werden (z.B. bei Temperaturen oberhalb 160° C).

Als Y kommen insbesondere der 4,4'-Diphenylmethanrest, der 2,4-Toluylenrest, der 3,5-Diisopropyl-3',5'-diethyl-4,4'-diphenylmethanrest, der 3,5-Diethyl-2,4 (35 % 2,6-Anteil)-toluylenrest und/oder der 1,4-phenylenrest in Frage.

Die dargestellten Anwendungs- oder Verarbeitungsbeispiele für die erfindungsgemäßen Dimer-diamine dienen lediglich zur Illustration einer mannigfaltig abwandelbaren Anwendung dieser neuen Diamine im Polyadditionsverfahren. Sie stellen jedoch bevorzugte Ausführungsformen dar.

Mit den erfindungsgemäßen Dimer-diaminen können nicht nur die als heterogene Diamin-Vernetzung bezeichneten Verfahren bei deutlich erhöhter Lagerstabilität, Schwerlöslichkeit und dennoch bei hoher Temperatur zu hochwertigen Elastomeren führenden Reaktionen durchgeführt werden, sondern es entstehen auch Elastomere, die bei Bedarf noch aus den bereits eingebauten Uretdiongruppen weiter vernetzt werden können. Diese Weitervernetzung kann durch eine Erhöhung der Temperatur z.B. auf 150° C, oder auch durch Einwirkung hochreaktiver (aliphatischer) Polyamine, z.B. durch Ethylendiamin oder Isophorondiamin, hervorgerufen werden, wobei an der Oberfläche außerordentlich hochvernetzte Systeme entstehen.

Bei Verwendung der erfindungsgemäßen Dimer-diamine können auch besonders geeignete aromatische

Diisocyanate, z.B. Diphenylmethan-4,4'-diisocyanate zum polymeraufbau verwendet werden, ohne daß dabei die Lagerstabilität der Gemische betroffen ist. Die Reaktionsverzögerung (Lagerstabilität) bei Verwendung der erfindungsge-mäßen Dimer-diamine ist erheblich größer als z.B. bei der Verwendung von Diaminen mit elektronenanziehenden, die Basizität der Aminogruppen vermindernden Substituenten, z.B. 3,3'-Dichlor-4,4'-diaminodiphenylmethan oder 4-Chlor-3,5-diamino-benzoesäure-isobutylester. Die Reaktionsverzögerung ist auch deutlich stärker als bei Anwendung von Komplexverbindungen mit Salzen und Aminen, die erst beim Erhitzen zerfallen und das reaktive Amin freigeben (US-PS 3 891 606).

Eine Kontrolle der Polyadditionsgeschwindigkeit kann auch durch Verwendung eines erst in der Hitze sich lösenden und wirksam werdenden Diisocyanats, Katalysators oder Vernetzers stattfinden, wie dies anhand konkreter Beispiele von H.w.Cox und S.A.Iobst in plast. Engn. 34 (1978), 5, S. 49 - 52 beschrieben ist. Die Verwendung schwerlöslicher und erst in der Sitze reagierender Diamine wird auch als heterogene Aminvernetzung bezeichnet und ist in DE-OS 2 635 400 beschrieben. Die neuartigen, erfindungsgemäßen Dimer-diamine zeigen darüber hinaus jedoch die bereits beschriebenen Vorteile, u.a. einen außerordentlich starren Aufbau und gegebenenfalls die Möglichkeit einer Selbstvernetzung.

Zur Herstellung der erfindungsgemäßen Dimer-diamine geeignete Uretdion-diisocyanate (dimere Diisocyanate) sind z.B. dimeres Toluylen-2,4-diisocyanat, dimeres 4,4'-Di-isocyanato-diphenylmethan oder seine oligo-dimeren, linearen Homologen, z.B. mit bis zu 4 Uretdionringen im Molekül, dimeres 2,4'-Diisocyanato-diphenylsulfid, dimeres 4,4'-Diisocyanato-diphenylsulfid, dimeres 4,4'-Diisocya-nato-diphenylsulfon, sowie alle anderen bekannten aromatischen Uretdiondiisocyanate und Mischungen aus solchen Uretdiondiisocyanaten. Bevorzugt sind dimeres Toluylen-2,4-diisocyanat und dimeres 2,4'- und/oder 4,4'-Diiso-cyanato-diphenylmethan.

Die Herstellung der Uretdion-diisocyanate aus den entsprechenden Diisocyanaten ist schon seit langer Zeit bekannt (vgl. Hofmann, Berichte 3 (1870), S. 765). Die Herstellung von z.B. dimerem Toluylen-2,4-diisocyanat ist im Kunststoffhandbuch - Band 7, polyurethane, herausgegeben von Vieweg/Höchtlen, Carl-Hanser-Verlag, München 1966, S. 16 ausführlich beschrieben. Sie gelingt durch Dimerisierung der obengenannten Diisocyanate mit Katalysatoren wie Trialkylphosphiten (DE-OS 2 349 726), peralkylierten Carbamoylphosphiten (US-PS 3 290 288), peralkylierten Aminophosphinen (U8-p8 3 290 288), 3- oder 4-substituier-ten pyridinen (GB-F8 821 158, 944 309, 962 689) wie z.B. 4-Dimethylaminopyridin, mit Trialkylphosphinen (luftempfindliche, hochreaktive Substanzen, DE-OS 2 420 475), Dialkylarylphosphinen und Alkyldiarylphosphinen (US-FS 2 671 082), Trialkylarsinen (Analytical Chemistry of the Polyurethanes, Bd. 16/III, High-polymers-Series (Wiley 1969), S. 112 - 131), Dibutylzinndilaurat (DE-OS 2 420 475) ohne Katalysator in einem Carboxylsäureester (USSR-PS 149 775) oder in wäßriger Emulsion (GB-PS 1 134 285).

Auch oligomere Uretdiondiisocyanate der allgemeinen Formel

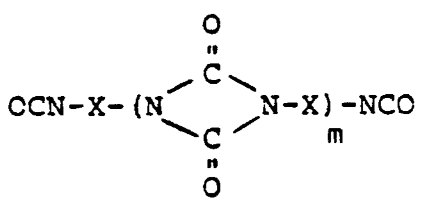 ,

worin X ein aromatischer Rest ist und m zwischen 1 und 10 liegt, können verwendet werden. Bevorzugt ist das oligomere Uretdion-diisocyanat aus 4,4'-Diphenylmethan-diiso-cyanat, welches bevorzugt bei niedrigen Temperaturen (z.B. 20° C) in wenig polaren Lösungsmitteln wie aliphatischen Kohlenwasserstoffen durch Zusatz von Katalysatoren hergestellt werden kann.

Geeignete aromatische Diamine $NH_2$-Y-$NH_2$ zur Herstellung der erfindungsgemäßen Dimer-diamine sind z.B. 3,5-Diethyl-2,4-diaminotoluol, 3,5-Diethyl-2,6-diaminotoluol und ihre Isomerengemische, 2,4-Diaminotoluol und/oder 2,6-Diamino-toluol, 4,4'- und/oder 2,4'-Diaminodiphenylmethan, in 3- und/oder 5-Stellung di- oder in 3,3',5,5'-Stellung tetraalkylierte ($C_1-C_4$-Alkyl)-diaminodiphenylmethane, p-phenylendiamin, m-phenylendiamin, 4,4'- und 2,4'-Diaminodi-phenylsulfid, 3,3'-Dimethylthio-4,4'-diaminodiphenylme-than oder Naphthylendiamin. Neben diesen Diaminen, bei denen X einen aromatischen Rest mit Molmassen von 76 bis 499 bedeutet, können auch höhermolekulare polyamine ($H_2$-Y-$NH_2$ mit Molmassen von oberhalb 500 bis 10 000, vorzugsweise 1000 bis 6000, verwendet werden. Derartige höhermolekulare aromatische Di- bzw. Polyamine sind beispielsweise durch Umsetzung von NCO-prepolymeren aus höhermolekularen Diolen, z.B. höhermolekularen polyetherdiolen oder polyesterdiolen und überschüssigen Mengen an aromatischen Diisocyanaten, vorzugsweise Toluylendiisocyanaten, durch Hydrolyse mit löslichen oder unlöslichen basischen Verbindungen, z.B. Alkalihydroxiden, niedermolekularen Natriumsilikaten, Alkalicarbonaten oder basischen Ionenaustauschern in überschüssigem wasser und in Gegenwart von wasserlöslichen Lösungsmitteln leicht zugänglich, z.B. nach dem Verfahren der DE-OS 2 948 419 und 3 039 600 und sind für das vorliegende Verfahren geeignete produkte.

Als inerte Lösungsmittel können z.B. aliphatische oder aromatische Kohlenwasserstoffe, Ether, Ester oder Ketone verwendet werden, z.B. höhersiedender Petrolether, Toluol, Dioxan, Ethylacetat, Methylethylketon; ferner Weichmacher wie phthal- oder Isophthalsäurealkylester, phosphorsäuretrisalkylester oder ähnliche

Verbindungen. Niedermolekulare polyole (mit Molmassen von 62 bis 399), bzw. höhermolekulare polyole (mit Molmassen von 400 bis etwa 10 000) sind übliche Verbindungen, wie sie für die Polyurethanherstellung bekannt sind.

Bei der Verwendung der neuartigen Dimer-diamine zum Aufbau von Polyurethanen können alle für die polyurethanherstellung beschriebenen Ausgangskomponenten wie höhermolekulare Polyhydroxy- oder Polyaminoverbindungen, niedermolekulare Kettenverlä gerungsmittel wie polyole oder polyamine und weitere Polyisocyanate, sowie übliche Zusatzund Hilfsmittel mitverwendet werden. Derartige geeignete Bubstanzen sind ausführlich in der DE-OS 2 854 384 beschrieben.

Von der Erfindung werden Dimer-diamine ausgenommen, bei welchen der Rest X der 2,4'-Disulfidrest ist, und wenn das Diamin $H_2N$-Y-$NH_2$ Molmassen von $\geqslant$ 500 aufweist. Derartige Verbindungen sind in der eigenen deutschen Patentanmeldung P 3 135 542.0 beschrieben.

Die entstandenen Dimerdiamine werden aus den Lösungsmitteln oder weichmachern im allgemeinen abfiltriert und als Pulver gewonnen. Diese Pulver können dann z.B. in den höhermolekularen Polyhydroxylverbindungen für die Polyurethansynthese suspendiert werden. Bevorzugt werden sie direkt in Suspension in den höhermolekularen Polyhydroxylverbindungen, bevorzugt weniger reaktiven Polyhydroxylverbindungen mit sekundären OH-Endgruppen (z.B. Polypropylenether-polyolen) hergestellt.

**Beispiele**

A) Allgemeine Verfahrensvorschrift für die Herstellung der Uretdion-diharnstoff-diamine

Die Uretdion-diharnstoff-diamine ("Dimer-diamine') werden hergestellt, indem man das aromatische Diamin in einem NCO-inerten Lösungsmittel wie z.B. Toluol löst oder suspendiert und die berechnete Menge des dimeren Diisocyanats (Uretdion-diisocyanats) in Molverhältnissen von etwa 2 Mol Diamin zu 1 Mol Uretdion-diisocyanat, (portionsweise) hinzugibt. Es entsteht bald eine dicke Suspension, die man noch 2 bis 8 Stunden bei 20 bis 110°C, vorzugsweise bei 40 bis 50°C nachrührt. wegen einer geringen Vorverlängerungsreaktion (d.h. einer Oligomerenbildung unter Verknüpfung zweier Diisocyanate durch ein Diamin) bleibt in der flüssigen Phase etwas überschüssiges aromatisches Diamin in vielen Fällen gelöst. Man wäscht dieses gegebenenfalls aus, saugt ab, trocknet und zerkleinert gegebenenfalls gebildete Agglomerate. Entsprechende Ergebnisse der Beispiele 1 bis 12 sind in Tabelle 1 wiedergegeben.

B) Herstellung der Abmischungen aus den erfindungsge-mäßen Uretdion-diharnstoff-diaminen und Polyester-Polyolen

Die in Tabelle 2 jeweils angegebene Menge des Uretdiondiharnstoff-diamins wird in der angegebenen Menge Polyester verrührt. In einem hochtourig drehenden Mischgerät wird so lange gerührt, bis eine feinteilige Suspension oder Paste entstanden ist. Vor der Verwendung wird die Suspension oder Paste bei 80°C eine Stunde lang entgast. Vergleiche Tabelle 2 für Beispiele 13 bis 24.

**Tabelle 1:** Uretdion-diharnstoff-diamine > 1 ("Dimer-diamine")

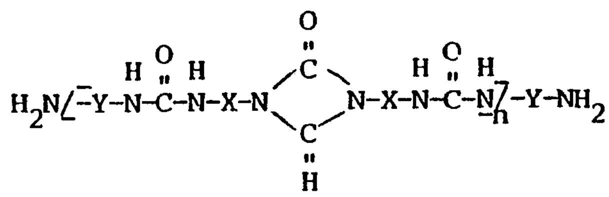

| Bsp. | Ausgangsverbindungen | | | | | | |
|---|---|---|---|---|---|---|---|
| Nr. | aromatisches Diamin | Uretdiondiisocyanat | Reaktions-temperatur /°C/ | Ausb. /%/ d.Th. | Molgewicht (MG) theor. | nach $HClO_4$-Titr. | Vorverlängerungsgrad n, aus MG berechnet |
| 1 | 3,5-Diethyl-2,4(35% 2,6-Anteil)-diaminotoluol | dimeres 2,4-Diisocyanatotoluol ("TT") | 20 | 78 | 704 | 901 | 1,37 |
| 2 | " | " | 40 | 90 | " | 1273 | 2,08 |
| 3 | " | " | 110 | 81 | " | 1061 | 1,68 |
| 4 * | " | " | 20 | 60 | ∞ | 1292 | 2,12 |
| 5 | 4,4'-Diaminodiphenylmethan | " | 40 | 85 | 744 | 1198 | 1,83 |
| 6 | 2,4-Diaminotoluol | " | 40 | 95 | 592 | 980 | 1,83 |
| 7 | 3,5-Diisopropyl-3',5'-diethyl-4,4'-diaminodiphenylmethan | " | 40 | 75 | 1024 | 1471 | 1,65 |

* eingesetztes Molverhältnis Diamin/TT = 1/1 (ein sehr hohes Molekulargewicht wäre theoretisch zu erwarten gewesen).

**Tabelle 1: Uretdion-diharnstoff-diamine** ("Dimer-diamine") (Fortsetzung)

| Bsp. Nr. | Ausgangsverbindungen: aromatisches Diamin | Ausgangsverbindungen: Uretdiondiisocyanat | Reaktionstemperatur /°C/ | Ausb. /%/ d.Th. | Molgewicht (MG) theor. | Molgewicht (MG) nach $HClO_4$-Titr. | Vorverlängerungsgrad n, aus MG berechnet |
|---|---|---|---|---|---|---|---|
| 8 | 3,5-Diethyl-2,4-(35% 2,6-Anteil)-diaminotoluol | dimeres 2,4'-Diisocyanatodiphenylsulfid | 40 | 98 | 892 | 1180 | 1,40 |
| 9 | 4,4'-Diaminodiphenylmethan | " | 40 | 98 | 932 | 1176 | 1,33 |
| 10 | 2,4-Diaminotoluol | " | 40 | 88 | 780 | 1000 | 1,33 |
| 11 | 3,5-Diisopropyl-3',5'-diethyl-4,4'-diaminodiphenylmethan | " | 40 | 69 | 1212 | 1361 | 1,17 |
| 12 | 3,5-Diethyl-2,4(35 % 2,6-Anteil)-diaminotoluol | dimeres 4,4'-Diisocyanatodiphenylmethan | 20 | 80 | 856 | 980 | 1,39 |

**Tabelle 2 zu B:** Abmischungen aus den erfindungsgemßen Uretdion-diharnstoff-diaminen und Polyester-Polyol

| Abmischung Beispiel Nr. | Uretdion-diharnstoff-diamin aus Tabelle 1, Beispiel Nr. | Abmischung bestehend aus g Aminkomponente | g Polyester aus Adipinsäure, Ethylenglykol, Butandiol-1,4 (1:1) MG 2000 |
|---|---|---|---|
| 13 | 1 | 15 | 85 |
| 14 | 2 | 15 | 85 |
| 15 | 3 | 15 | 85 |
| 16 | 4 | 15 | 85 |
| 17 | 5 | 15 | 85 |
| 18 | 6 | 15 | 85 |
| 19 | 7 | 15 | 85 |
| 20 | 8 | 16,7 | 75 |
| 21 | 9 | 14,5 | 75 |
| 22 | 10 | 14,6 | 75 |
| 23 | 11 | 22,7 | 75 |
| 24 | 12 | 15,0 | 85 |

C) Verwendung der Uretdion-diharnstoff-diamine zur Herstellung von Elastomeren
In den folgenden Beispielen werden NCO-Prepolymere verwendet, die alle nach der in Beispiel 25 a) aufgeführten Herstellungsvorschrift gewonnen wurden.

**Beispiel 25**

a) Herstellung der NCO-Prepolymeren
2000 g (1 mol) eines Polyesters aus Adipinsäure und Ethylenglykol werden 1 Stunde bei 100° C entwässert. 348 g (2 mol) eines Gemischs aus 80 % 2,4-Diisocyanatotoluol und 20 % 2,6-Diisocyanatotoluol werden in einem Guß zugegeben und die Mischung bei 80° C gerührt, bis der theoretische End-NCO-Gehalt von 3,58 % erreicht ist.
b) Elastomerherstellung
152,5 g des nach a) gewonnenen NCO-Prepolymeren werden bei 110° C 20 Minuten lang entgast. 100 g der nach B), Beispiel Nr. 15 hergestellten Paste werden 1 Stunde bei 50° C entgast. Beide Komponenten werden miteinander gemischt und bei 100° C in eine auf unter 110° C vorgeheizte Form gegossen. Das Gemisch besitzt bei 100° C eine Gieß-zeit von 12 Minuten und ist bei 40° C praktisch unbegrenzt lagerstabil.
Nach 24-stündigem Ausheizen der in der Form befindlichen Masse erhält man ein hochelastisches Material mit den in Tabelle 3 aufgeführten mechanischen Eigenschaften.

**Vergleichsbeispiele:**

Die folgenden Vergleichsbeispiele werden herangezogen um zu zeigen, daß die guten mechanischen Eigenschaften der mit den erfindungsgemäßen Dimer-diaminen vernetzten Elastomeren nicht auch schon durch Verlängerung und Vernetzung mit äquimolaren Mengen homogener Aminvernetzer oder mit Polyol alleine erreicht werden:

**Vergleichsbeispiel 1)**

Zur Herstellung von Vergleichselastomeren mit homogener Diaminvernetzung werden anstelle des erfindungsgemäßen heterogenen Dimer-diamins gleiche molare Mengen des zugrunde liegenden aromatischen Diamins verwendet: 2,98 g 3,5-Diethyl-2,4-(35 % 2,6-Anteil)-diaminotoluol werden in 89,2 g Polyester aus Adipinsäure, Ethylenglykol/Butan-diol-1,4 (1:1), Molekularmasse 2000, gelöst und mit 160 g des nach a gewonnenen NCO-Prepolymers wie in Beispiel 25 beschrieben vernetzt.

**Vergleichsbeispiel 2)**

Zur Herstellung eines Vergleichselastomeren ohne Diaminvernetzung werden 170 g des nach a) gewonnenen NCO-Prepolymers ohne einen Diamin-Kettenverlängerer nur mit 142,4 g des obengenannten Polyesters aus Adipinsäure-Ethylenglykol/Butandiol-1,4 (Molverhältnis 1:1), wie in b) beschrieben, zu einem Elastomeren umgesetzt.

Die Herstellung von Vergleichs-Gießelastomeren aus denselben Ausgangskomponenten, aber in herkömmlicher Reihenfolge der Reaktionsteilschritte ist wegen der extrem kurzen Topfzeit von $^1/_2$ min oder weniger bei 80° C nicht möglich, wie Vergleichsbeispiel 3 zeigt:

**Vergleichsbeispiel 3)**

Anstelle des fertigen Adduktes werden dimeres Toluylendiisocyanat und 3,5-Diethyl-2,4(2,6 -65/35)-toluylendiamin getrennt an geeigneter Stelle dem Elastomer-Gieß-ansatz zugeführt: 7,42 g dimeres Toluylendiisocyanat werden in 152,5 g des nach 25 a) erhaltenen NCO-Prepolymers gelöst. 15 g des Diamins werden in 85 g Polyester nach Tabelle 2 (Adipinsäure, Ethylenglykol/Butandiol-1,4 (1:1) Polyestermolekulargewicht 2000) gelöst und mit dem das dimere Toluylendiisocyanat gelöst enthaltenden NCO-Prepolymer bei 80° C versetzt. Das Elastomer kann bei außer-ordentlich raschem, nur auf labormäßige Durchführung beschränktem Arbeiten zu einem Elastomer verarbeitet werden, das dem äußeren Anschein nach vergleichbare mechanische Eigenschaften aufweist wie das Elastomer aus Beispiel 25, das jedoch eine Gießzeit von 15 min bei 80° C besitzt. Ein Gießelastomer-Formkörper kann jedoch nach Vergleichsbeispiel 3 nicht hergestellt werden, weil die Polyadditionsreaktion viel zu rasch läuft. Vergleichsbeispiel 3) gegen Beispiel 25 b) zeigt deutlich die Vorzüge der heterogen vernetzenden Voraddukte aus Uretdiondiisocyanaten und aromatischen Diaminen zur Herstellung hochwertiger Polyurethan-Elastomerer gegenüber der Vernetzung mit homogen gelösten Reaktionspartnern (hier dimeres Toluylendiisocynat und das Diethyltoluylendiamin). Vergleiche auch Tabelle 3.

**Tabelle 3:** Mechanische Eigenschaften des Elastomeren aus Beispiel 25 und der Elastomeren aus Vergleichsbeispiel 1 und 2

| | 25 | Vgl. 1 | Vgl. 2 |
|---|---|---|---|
| Gießzeit bei 80°C [min] | 15 | 2 | 8 |
| Entformungszeit [min] | 60 | 10 | 60 |
| Zugfestigkeit [MPa]<br>DIN 53 504 | 36,9 | 12,6 | 4,8 |
| Reißdehnung [%]<br>DIN 53 504 | 606 | 805 | 256 |
| Weiterreißfestigkeit nach [KN/m]<br>DIN 53 515 | 22,7 | 17 | 15 |
| Shore-Härte nach A<br>DIN 53 505 D | 78<br>26 | 77<br>25 | 68 *<br>18 |
| Elastizität nach [%]<br>DIN 53 512 | 46 | 39 | 33 |
| Druckverformungsrest nach<br>DIN 53 517, 24 h bei 70°C [%] | 13 | 64 | bei 70°C<br>Verflüssigung |

*) Die Shore-A-Härte beruht hier im wesentlichen auf der Kristallinität des Polyesters, wie sich durch die Verflüssigung bei der Messung des Druckverformungsrestes bei 70°C herausstellt.

**Beispiel 26**

162 g Prepolyner von Beispiel 25 werden analog Beispiel 25 mit 100 g Paste Beispiel Nr. 18 versetzt.

**Veigleichsbeispiel 4**

160 g Prepolyer von Beispiel 25 werden analog Vergleichsbeispiel 1 mit 83 9 g Polyester und 3,05 g 2,4-Dianinotoluol versetzt. Tabelle 4: Mechanische Eigenschaften des Elastomeren aus Beispiel 26 und des Elastomeren aus Vergleichsbeispiel 4

| | 26 | Vgl. 4 |
|---|---|---|
| Gießzeit bei 80°C [min] | 10 | 2 |
| Entformungszeit [min]<br>DIN 53 504 | 60 | 12 |
| Zugfestigkeit [MPa]<br>DIN 53 504 | 30,8 | 17,9 |
| Reißdehnung [%] | 678 | 794 |
| Weiterreißfestigkeit nach<br>DIN 53 515 [KN/m] | 27,4 | 29,9 |
| Shore-Härte nach A<br>DIN 53 505 D | 71<br>23 | 83<br>30 |
| Elastizität nach<br>DIN 53 512 [%] | 47 | 35 |
| Druckverformungsrest nach<br>DIN 53 517, 24 h bei 70°C [%] | 46 | 35 |

**Beisipiel 27**

135,3 g Prepolymer von Beispiel 25 werden analog Beispiel 25 mit 97,7 g Paste Beispiel Nr. 23 versetzt.

**Vergleichsbeispiel 5**

160 g Prepolymer von Beispiel 25 werden analog Vergleichsbeispiel 1 mit 99,7 g Polyester und 5,81 g 3,5-Diisopropyl-3',5'-dietyl-4,4'-diaminodiphenylmethan versetzt. Tabelle 5: Mechanische Eigenschaften des Elastomeren aus Beispiel 27 und des Elastomeren aus Vergleichsbeispiel 4

| | 27 | Vgl. 5 |
|---|---|---|
| Gießzeit bei 80°C [min] | 7 | 1,5 |
| Entformungszeit [min] | 120 | 5 |
| Zugfestigkeit [MPa]<br>DIN 53 504 | 22,1 | 10,6 |
| Reißdehnung [%]<br>DIN 53 504 | 620 | 794 |
| Weiterreißfestigkeit nach<br>DIN 53 515 [KN/m] | 20,3 | 18,4 |
| Shore-Härte nach A<br>DIN 53 505 D | 77<br>28 | 82<br>29 |
| Elastizität nach<br>DIN 53 512 [%] | 48 | 42 |
| Druckverformungsrest nach<br>DIN 53 517, 24 h bei 70°C [%] | 13 | 74 |

**Beispiel 28**

143,2 g Prepolymer von Beispiel 25 werden analog Beispiel 25 mit 100 g Paste Beispiel Nr. 24 versetzt. Vergleichsbeispiel: identisch Vergleichsbeispiel 1

**Tabelle 6:** Mechanische Eigenschaften des Elastomeren aus Beispiel 28 und des Elastomeren aus Vergleichsbeispiel 1

| | 28 | Vgl. 1 |
|---|---|---|
| Gießzeit bei 80 °C [min] | 11 | 2 |
| Entformungszeit [min] | 80 | 10 |
| Zugfestigkeit [MPa] DIN 53 504 | 13,2 | 12,6 |
| Reißdehnung [%] DIN 53 504 | 708 | 805 |
| Weiterreißfestigkeit nach DIN 53 515 [KN/m] | 17,7 | 17 |
| Shore-Härte nach DIN 53 505 A | 79 | 77 |
| D | 27 | 25 |
| Elastizität nach DIN 53 512 [%] | 51 | 39 |
| Druckverformungsrest nach DIN 53 517, 24 h bei 70 °C [%] | 14 | 64 |

**Beispiele zur Verwendung der erfindungsgemäßen Amine in Abmischung mit homogen löslichen Amin-Vernetzern**

Die folgenden Beispiele werden herangezogen, um zu zeigen, daß die erfindungsgemäßen Amine in Polyether-Polyurethanen einen Teil des homogenen Aminvernetzers ersetzen können. Man erhält Elastomere mit einem insgesamt deutlich verbesserten Eigenschaftsniveau.

**Herstellung des NCO-Prepolymeren für Beispiele 29 und 30**

200 g (0,1 mol) lineares, bifunktionelles Polypropylenglykol, MG 2000, werden 1 h bei 100° C entwässert. 34,8 g (0,2 mol) 2,4-Diisocyanatotoluol werden zugegeben und die Mischung bei 80° C gerührt, bis der theoretische NCO-Gehalt von 3,58 % erreicht ist.

**Beispiel 29**

230,8 g des soeben beschriebenen Prepolymeren werden bei 110° C 20 min lang entgast.

Bei 80° C wird eine Mischung aus 13,6 g Diethyltoluylendiamin und 13,44 g des heterogenen Amins nach Beispiel 3 (aus dimerem Toluylendiisocyanat und 2 Mol Diethyltoluylendiamin) zugegeben und die Mischung in eine auf 110° C vorgeheizte Form gegossen. Nach 24-stündigem Ausheizen bei 110° C erhält man ein elastisches Material mit den in Tabelle 7 aufgeführten mechanischen Eigenschaften.

**Vergleichsbeispiel 6**

230,8 g des genannten Prepolymerswerden entgast und bei 80° C mit 17,0 g Diethyltoluylendiamin bei 80° C vernetzt. Die mechanischen Eigenschaften des resultierenden Elastomers sind in Tabelle 7 aufgeführt.

**Tabelle 7:** Mechanische Eigenschaften des Elastomeren aus Beispiel 29 und des Elastomeren aus Vergleichsbeispiel 6

| | 28 | Vgl. 6 |
|---|---|---|
| Gießzeit (80°C) [min] | 2 | 2 |
| Entformungszeit (110°C) [min] | 4 | 6 |
| Zugfestigkeit [MPa] DIN 53 504 | 7,5 | 7,5 |
| Reißdehnung [%] DIN 53 504 | 750 | 950 |
| Weiterreißfestigkeit nach DIN 53 515 [KN/m] | 25 | 21 |
| Shore-Härte nach DIN 53 505 A | 82 | 73 |
| D | 25 | 19 |
| Elastizität nach DIN 53 512 [%] | 56 | 54 |
| Druckverformungsrest nach DIN 53 517, 24 h bei 70°C [%] | 84 | 91 |

**Beispiel 30**

230,8 g des genannten Prepolymers werden entgast und bei 80°C mit einer Mischung aus 17,44 g geschmolzenem 3,5-Di-amino-4-chlorbenzoesäureisopropylester und 13,44 g des heterogenen Amins nach Beispiel 3 wie in Beispiel 29 beschrieben versetzt.

**Vergleichsbeispiel 7**

230,8 g des genannten Prepolymers werden wie beschrieben bei 80°C mit 21,8 g 3,5-Diamino-4-chlorbenzoesäureisopropylester versetzt. Tabelle 8: Mechanische Eigenschaften des Elastomeren aus Beispiel 30 und des Elastomeren aus Vergleichsbeispiel 7

| | 29 | Vgl. 7 |
|---|---|---|
| Gießzeit (110°C) [min] | 200 | 30 |
| Entformungszeit (110°C) [min] | 200 | 45 |
| Zugfestigkeit [MPa] DIN 53 504 | 6 | 4,8 |
| Reißdehnung [%] DIN 53 504 | 485 | 420 |
| Weiterreißfestigkeit nach DIN 53 515 [KN/m] | 35 | 19 |
| Shore-Härte nach DIN 53 505 A | 81 | 78 |
| D | 27 | 19 |
| Elastizität nach DIN 53 512 [%] | 45 | 42 |
| Druckverformungsrest nach DIN 53 517, 24 h bei 70°C [%] | 52 | 65 |

**Patentansprüche:**

1. Aromatische Uretdion-diharnstoff-diamine der Formel

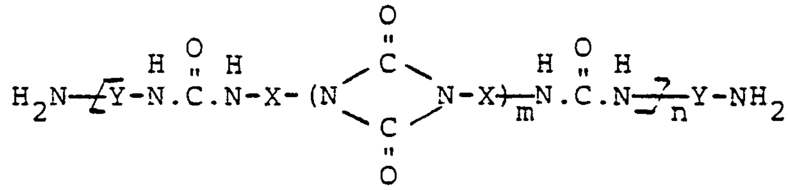

worin
X aromatische Reste mit Molmassen von 76 bis 499,
Y aromatische Reste wie X oder Reste

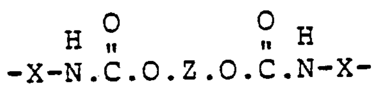

eines höhermolekularen Diamins darstellen, wo

Z der Rest von höhermolekularen und/oder niedermolekularen Dihydroxyverbindungen HO-Z-OH ist,

m der mittlere Uretdionisierungsgrad eine Zahl zwischen 1 und 10 ist und

n der mittlere Vorverlängerungsgrad zwischen 1 und 3 liegt

und wobei Diamine ausgeschlossen sind, bei welchen X der 2,4'-Diphenylsulfid-Rest ist, wenn das Diamin $H_2N$-Y-$NH_2$ Molmassen von ⩾ 500 besitzt.

2. Aromatische Uretdion-diharnstoff-diamine nach Anspruch 1, dadurch gekennzeichnet, daß die Reste X den 2,4-Toluylenrest, den 4,4'-Diphenylmethanrest und/oder den 2,4'-Diphenylmethanrest darstellen.

3. Aromatische Uretdion-diharnstoff-diamine nach Ansprüchen 1 und 2, dadurch gekennzeichnet, daß m Werte zwischen 1 bis 2 darstellt.

4. Aromatische Uretdion-diharnstoff-diamine nach Ansprüchen 1 bis 3, dadurch gekennzeichnet, daß n Werte zwischen 1 und 3 darstellt.

5. Aromatische Uretdion-diharnstoff-diamine nach Ansprüchen 1 bis 4, dadurch gekennzeichnet, daß die Reste Y den 4,4'-Diphenylmethanrest, den 3,5-Di-isopropyl-3',5'-diethyl-4,4'-diphenylmethanrest, den 3,5-Diethyl-2,4-(35 % 2,6-)toluylenrest und/ oder den 1,4-Phenylenrest darstellen.

6. Aromatische Uretdion-diharnstoff-diamine nach Ansprüchen 1 bis 4, dadurch gekennzeichnet, daß die höhermolekularen Diamine $H_2N$-Y-N $H_2$ eine Molmasse von 1000 bis 6000 aufweisen.

7. Verfahren zur Herstellung von aromatischen Uretdion-diharnstoff-diaminen der Formel

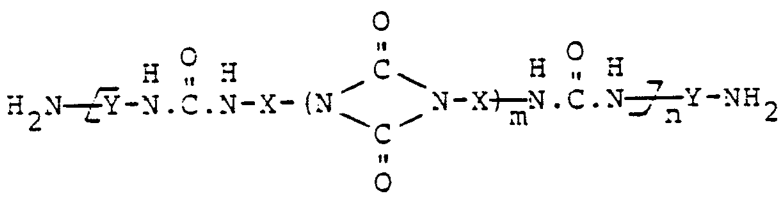

in feinteiliger, gegebenenfalls suspendierter Form durch Vermischen einer Lösung oder Suspension eines aromatischen Diamins $H_2$N-Y-N$H_2$ in einem gegenüber Isocyanaten inerten Lösungsmittel, Weichmacher oder einem höhermolekularen Polyol

mit einem feinteiligen, gegebenenfalls in einem gegenüber Isocyanaten inerten Lösungsmittel, Weichmacher und/oder einen höhermolekularen Polyol suspendierten Uretdion-diisocyanat

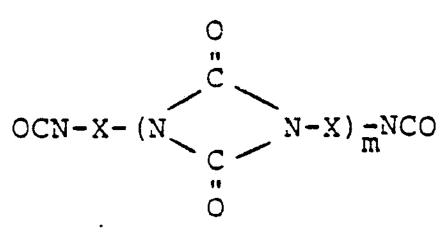

wobei das Molverhältnis von Diamin zu Diisocyanat 1,8:1 ist und

X aromatische Reste mit Molmassen von 76 bis 499,

und

Y aromatische Reste wie X oder Reste

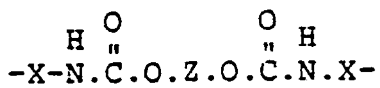

darstellen, wo
X der Rest von höhermolekularen und/oder niedermolekularen Dihydroxyverbindungen HO-Z-OH ist,
n der mittlere Vorverlängerungsgrad mit Zahlen zwischen 1 und 3 ist
und
m der mittlere Uretdionisierungsgrad mit Zahlen zwischen 1 und 10 ist,

wobei die Herstellung solcher aromatischer Uretdion-diharnstoff-diamine ausgeschlossen ist, bei welchen X der 2,4'-Diphenylsulfidrest ist und das Diamin $H_2N-Y-NH_2$ Molmassen von $\geqslant$ 500 aufweist.

8. Verwendung der feinteiligen Uretdion-diharnstoffdiamine, nach Ansprüchen 1 bis 6, gegebenenfalls suspendiert in höhermolekularen Polyhydroxylverbindungen, als Aufbaukomponente zur Herstellung von Harnstoffgruppen-haltigen, hochmolekularen, gegebenenfalls über die Uretdiongruppen vernetzbaren Polyadditionsprodukten.

9. Verwendung der Uretdion-diharnstoff-diamine entsprechend Anspruch 8, dadurch gekennzeichnet, daß man die feinteiligen Uretdion-diharnstoffdiamine, gegebenenfalls suspendiert in höhermolekularen Polyhydroxylverbindungen, zusammen mit NCO-Prepolymeren aus Polyolen und überschüssigen Polyisocyanaten und/oder zusammen mit Polyisocyanaten,

sowie gegebenenfalls weiteren höhermolekularen Polyhydroxylverbindungen der Molmasse 400 bis 10 000 und gegebenenfalls niedermolekularen Kettenverlängerungsmitteln, besonders Diolen oder aromatischen Diaminen der Molmasse 62 bis 399,

bei Temperaturen oberhalb 100° C

und einem Äquivalentverhältnis zwischen NCO-Gruppe und der Summe von OH- und NH -Gruppen zwischen 0,9 und 1,4 umsetzt und gegebenenfalls bei Anwesenheit überschüssiger OH- und/oder $NH_2$-Gruppen bei Temperaturen oberhalb 140° C vernetzt.

10. Verwendung der Uretdion-diharnstoff-diamine entsprechend Anspruch 8, dadurch gekennzeichnet, daß man Umsetzungsprodukte von langkettigen Diaminen $H^2N$-Y-$NH^2$ mit einer Molmasse $\geqslant$ 500 mit Uretdiondiisocyanaten, gegebenenfalls in Anwesenheit von weiteren, niedermolekularen aromatischen Diaminen bei Temperaturen oberhalb 140° C als selbstvernetzende Einkomponentensysteme einsetzt.

**Claims**

1. Aromatic uretdione diurea diamines of the formula

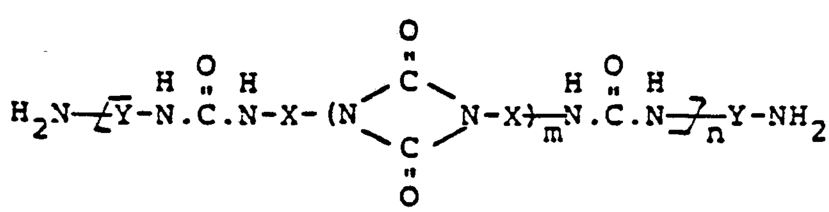

wherein
X represents aromatic radicals baving molecular weights of 76 to 499,
Y represents the same aromatic radicals as X or radicals

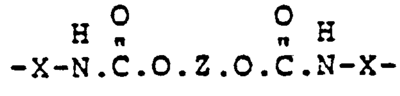

of a higher molecular weight diamine, where
Z is the radical of higher molecular weight and/or low molecular weight dibydroxy compounds HO-Z-OH,
m is the average degree of uretdionisation and is a number betw 1 and 10, and
n is tbe average degree of pre-extension and is between 1 and 3 diamines being excluded in which X is the

2,4'-diphenylsulphide radical, when the diamine $H_2N$-Y-$NH_2$ bas molecular weights of ⩾ 500.

2. Aromatic uretdione diurea diamines according to Claim 1, characterised in that the radicals X represent the 2,4-toluylene radical, the 4,4'-diphenylmethane radical and/or the 2,4'-diphenylmethane radical.

3. Aromatic uretdione diurea diamines according to Claims 1 and 2, characterised in that m represents values between 1 and 2.

4. Aromatic uretdione diurea diamines according to Claims 1 to 3, characterised in that n represents values between 1 and 3.

5. Aromatic uretdione diurea diamines according to Claims 1 to 4, characterised in that the radicals Y represent the 4,4'-diphenylmethane radical, the 3,5-diisopropyl-3',5'-diethyl-4,4'-diphenylmethane radical the 3,5-diethyl-2,4-(35% 2,6-)toluylene radical and/or the 1,4-phenylene radical.

6. Aromatic uretdione diurea diamines according to Claims 1 to 4 characterised in that the higher molecular weight diamines $H_2N$-Y-$NH_2$ have a molecular weight of 1,000 to 6,000.

7. Process for the preparation of aromatic uretdione diurea diamines of the formula

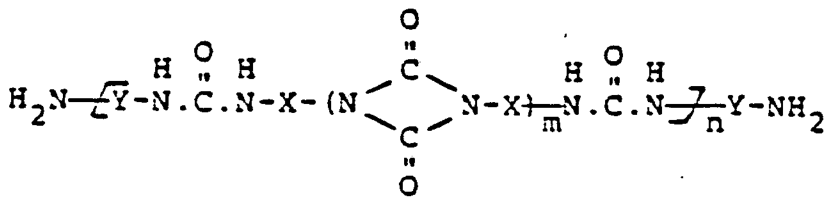

in a finely divided, optionally suspended form, by mixing a solution or suspension of an aromatic diamine $H_2H$-Y-$NH_2$ in an isocyanate-inert solvent, plasticiser or in a higher molecular weight polyol, with a finely-divided uretdione diisocyanate

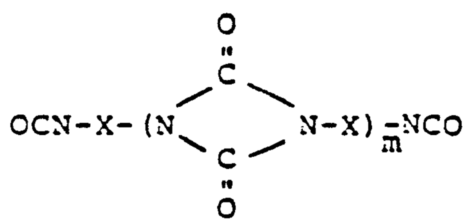

optionally suspended in an isocyanate-inert solvent, plasticiser and/or in a higher molecular weight polyol, wherein the molar ratio of diamine to diisocyanate is 1.8:1 and
X represents aromatic radicals with molecular weights of 76 to 499 and
Y represents the same aromatic radicals as X or radicals

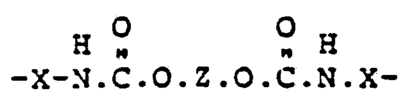

where
X is the radical of higher molecular weight and/or low molecular weight dihydroxy compounds HO-Z-OH,
n is the average degree of pre-extension and is a number between 1 and 3 and
m is the average degree of uretdionisation and is a number between 1 and 10,
the production of those aromatic uretdione diurea diamines being excluded in which X is the 2,4'diphenylsulphide radical and the diamine $H_2N$-Y-$NH_2$ has molecular weights of ⩾ 500.

8. Use of the finely divided uretdione diurea diamines, according to Claims 1 to 6, optionally suspended in higher molecular weight polyhydroxyl compounds, as a starting component for the production of urea-group-containing, high molecular weight polyaddition products which are optionally crosslinkable via the uretdione groups.

9. Use of the uretdione diurea diamines according to Claim 8, characterised in that the finely divided uretdione diurea diamines, optionally suspended in higher molecular weight polyhydroxyl compounds, are reacted together with NCO prepolymers of polyols and excess polyisocyanates and/or together with polyisocyanates, and optionally other higher molecular weight polyhydroxyl compounds of a molecular weight of 400 to 10,000 and optionally low molecular weight chainlengthening agents, in particular diols or aromatic diamines of a

molecular weight of 62 to 399, at temperatures higher than 100°C and with an equivalent ratio between the NCO group and the sum of OH and $NH_2$ groups of between 0.9 and 1.4 and the product is optionally crosslinked in the presence of excess OH and/or $NH_2$ groups at temperatures higher than 140°C.

10. Use of the uretdione diurea diamines according to Claim 8, characterised in that products of the reaction of long-chain dismines $H_2N$-Y-$NH_2$ having a molecular weight of ⩾ 500 with uretdione diisocyanates, optionally in the presence of other, low molecular weight aromatic diamines at temperatures higher than 140°C, are used as self-crosslinki one-component systems.

## Revendications

1. Uretdione-diurée-diamines aromatiques de formule

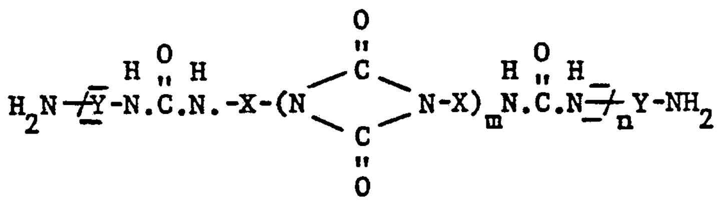

dans laquelle
X représente des restes aromatiques ayant des masses molaires de 76 à 499,
Y représente des restes aromatiques comme X ou des restes

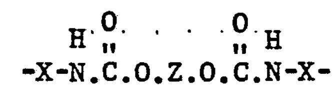

d'une diamine à poids moléculaire élevé où
Z est le reste de composés dihydroxylés HO-Z-OH à poids moléculaire élevé et/ou à bas poids moléculaire
m est le degré moyen de transformation en uretdione et représente un nombre compris entre 1 et 10 et
n est le degré moyen d'allongement préliminaire et représente un nombre compris entre 1 et 3
à l,exclusion des diamines pour lesquelles X est le reste 2 4'-sulfure de diphényle quand la diamine $H_2N$-Y-$NH_2$ possède des masses molaires égales ou supérieures à 500.

2. Uretdione-diurée-diamines aromatiques selon la revendication 1 caractériséesen ce que les restes X représentent le reste 2 4-toluylène le reste 4 4'-diphénylméthane et/ou le reste 2 4'-diphénylméthane.

3. Uretdione-diurée-diamines aromatiques selon les revendications 1 et 2 caractérisées en ce que m représente des valeurs comprises entre 1 et 2.

4. Uretdione-diurée-diamines aromatiques selon les revendications 1 à 3 caractérisées en ce que n représente des valeurs comprises entre 1 et 3.

5. Uretdione-diurée-diamines aromatiques selon les revendications 1 à 4 caractérisées en ce que les restes Y représentent le reste 4,4'-diphénylméthane, le reste 3,5-diisopropyl-3',5'-diéthyl-4,4'-diphénylméthane le reste 3,5-diéthyl-2,4-(35 % de 2,6)-toluylène et/ou le reste 1,4-phénylène.

6. Uretdione-diurée-diamines aromatiques selon les revendications 1 à 4, caractérisées en ce que les diamines $H_2N$-Y-$NH_2$ à poids moléculaire élevé présentent une masse molaire de 1000 à 6000.

7. Procédé de préparation d'uretdione-diuréediamines aromatiques de formule:

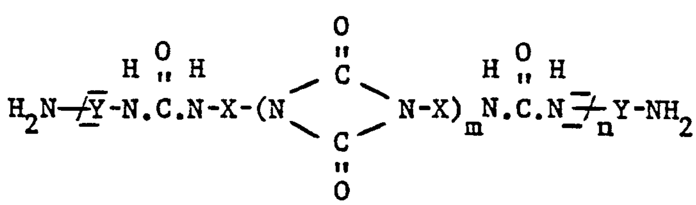

en fines particules éventuellement sous forme de suspension, par mélangeage d'une solution ou suspension d'une diamine aromatique $H_2N$-Y-$NH_2$ dans un solvant inerte à l'égard des diisocyanates, d'un plastifiant ou d'un polyol à poids moleculaire élevé, avec un diisocyanate d'uretdione

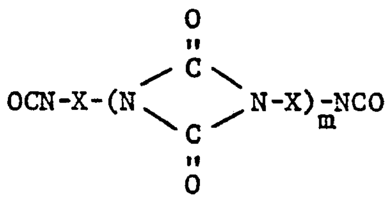

(en fines particules éventuellement en suspension dans un solvant inerte à l'égard des isocyanates, un plastifiant et/ou un polyol à poids moléculaire élevé), le rapport molaire de la diamine aux diisocyanates étant de 1,8:1, et

X représentant des restes aromatiques ayant des masses molaires de 76 à 499 et

Y représentant des restes aromatiques comme X ou des restes

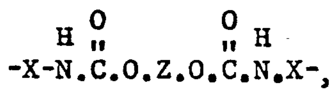 où

X est le reste de composés dihydroxylés HO-Z-OH à poids moléculaire élevé et/ou à bas poids moléculaire,

n est le degré moyen d'allongement préliminaire qui représente des nombres compris entre 1 et 3, et

m est le degré moyen de transformation en uretdione et représente des nombres compris entre 1 et 10,

à l'exclusion de la préparation d'uretdione-diurée-diamines aromatiques pour lesquelles X est le reste 2,4'-sulfure de diphényle et la diamine $H_2N$-Y-$NH_2$ présente des masses molaires égales ou supérieures à 500.

8. Utilisation des uretdione-diurée-dismines en fines particules selon les revendications 1 à 6 éventuellement en suspension dsns des composés polyhydroxylés à poids moléculaire élevé comme composants d'édification pour la préparation de produits de polyaddition contenant des groupes urées, à poids moléculaire élevé éventuellement réticulés par l'intermédiaire des groupes uretdiones.

9. Utilisation des uretdione-diurée-diamines selon la revendication 8 caractérisée en ce qu'on fait réagir les uretdione-diurée-diamines en fines particules, éventuellement en suspension dans des composés polyhydroxyles à poids moléculaire élevé,

avec des prépolymeres contenant des groupes NCO, obtenus à partir de polyols et de polyisocyanates en excès et/ou avec des polyisocyanates,

ainsi qu'éventuellement d'autres composés polyhydroxylés à poids moléculaire élevé de masse molaire 400 à 10000 et éventuellement des agents à bas poids d'allongement des chaînes notamment des diols ou des diamines aromatiques de masse-molaire de 62 à 399, à des températures supérieures à 100°C et selon un rapport entre équivalents de groupes NCO et de la somme des groupes OH et $NH_2$ compris entre 0,9 et 1,4 et éventuellement on réticule à des température supérieures à 140°C en présence d'un excès de groupes OH et/ou $NH_2$.

10. Utilisation des uretdione-diurée-diamines selon la revendication 8, caractérisée en ce qu'on utilise à des températures supérieures à 140°C comme système à autoréticulation à un composant, des produits de réaction de diamines $H_2N$-Y-$NH_2$ à longues chaînes ayant une masse molaire égale ou supérieure à 500 avec des diisocyanates d'uretdione, éventuellement en présence d'autres diamines aromatiques à bas poids moléculaire.